# EUROPÄISCHE PATENTSCHRIFT

(11) **EP 3 302 368 B1**
(45) Veröffentlichungstag und Bekanntmachung des Hinweises auf die Patenterteilung: **13.10.2021**
(21) Anmeldenummer: 16728368.8
(22) Anmeldetag: 23.05.2016
(51) Int. Cl.: A61F 2/28, A61B 17/68, A61F 2/30

(54) **GEWEBEDEHNUNGSVERSTÄRKER**
TISSUE EXPANSION BOOSTER
RENFORT POUR DILATATION DE TISSU

(30) Priorität: 03.06.2015 DE 202015004095 U
(43) Veröffentlichungstag der Anmeldung: 11.04.2018
(73) Patentinhaber: Perren, Nicolas, 4142 Münchenstein (CH)
(72) Erfinder: Perren, Nicolas, 4142 Münchenstein (CH)
(74) Vertreter: Dennemeyer & Associates S.A.
(86) Internationale Anmeldenummer: PCT/IB2016/000710
(87) Internationale Veröffentlichungsnummer: WO 2016/193801

(56) Entgegenhaltungen:
- WO-A1-2012/045307
- DE-A1-102012 202 750
- US-A1- 2005 113 924

## Beschreibung

Die Erfindung bezieht sich auf ein Implantat, insbesondere auf einen Gewebedehnungsverstärker gemäss dem Oberbegriff des Anspruchs 1.

Der Knochen stützt die Elemente des Bewegungsapparats und ermöglicht so dessen Funktion. Durch Überlast gebrochen verliert der Knochen seine mechanische Funktion. Die Reparatur der Funktion des gebrochenen Knochens, die Knochenbruch-Heilung, bedingt eine solide Überbrückung des Bruchs in anatomisch korrekter Lage. Eine erfolgreiche Knochenbruch-Heilung muss induziert, ermöglicht und unterhalten werden. Dabei spielen die mechanischen Bedingungen eine entscheidende Rolle. Dies gilt nicht nur für die Knochenbruch-Heilung sondern auch für Gewebe-Heilung allgemein wie auch für die Herstellung von Geweben im oder ausserhalb des menschlichen Körpers (z. B. tissue engineering).

### Definitionen:

Dehnung (Formelzeichen ε): Unter Dehnung wird im nachfolgenden eine Angabe für die relative Längenänderung (Verlängerung oder Verkürzung) in verschiedenen Richtungen eines Körpers unter Belastung, beispielsweise durch eine Kraft verstanden. Wenn sich die Abmessung des Körpers vergrössert wird dies als positive Dehnung bezeichnet, andernfalls als negative Dehnung oder Stauchung.

Kritisches Element der Mechanik in Bezug auf die Knochenheilung ist die GewebeDeformation (Dehnung ε im Sinne der Verlängerung und der Verkürzung, in verschiedenen Richtungen). Die Heilung wird einerseits angeregt, wenn die Gewebedehnung grösser als ein Mindestbetrag ist. Bei zu geringer Dehnung wird der Heilungsprozess nicht ausgelöst. Die Überbrückung des Bruchs ist andererseits nur möglich, wenn die vorliegende Dehnung des Gewebe-Elements nicht grösser als die Bruchelongation des reparierenden Gewebes ist. Zwischen diesen beiden Bedingungen, der Induktion und der Toleranz, besteht eine Bandbreite der Dehnung, die Voraussetzung für eine speditive und zuverlässige Heilung ist. Liegt die interfragmentäre Dehnung ausserhalb der erwähnten Bandbreite ist die Heilung gestört. Es entstehen Heilungsverzögerungen oder Pseudoarthrosen. Die Behandlung derartiger Heilungsstörungen bedingt eine Korrektur der Gewebedehnung. Durch Vergrösserung oder Verkleinerung kann die Dehnung in die erwähnte Bandbreite verändert und damit die Heilung ermöglicht werden.

Eine Knochenverankerung- oder Verbindungseinrichtung ist aus dem Dokument WO 2012/045307 bekannt. Diese bekannte Vorrichtung stellt eine Konstruktion dar bei der die beiden Endplatten starr miteinander verbunden. Es handelt sich somit um ein tragendes Verbindungselement, welches der Stabilisierung der überbrückten Knochenfragmente dient. DE 10 2012 202750 betrifft eine dynamische Stabilisierungseinrichtung für Knochen, insbesondere für die Wirbelsäule umfassen ein Stabilisierungssystem, insbesondere für die Wirbelsäule. US 2005/113924 A1 betrifft eine Zwischenwirbelprothese zur Implantation in eine Wirbelsäule umfassend ein starres Fixierungselement und ein komprimierbares Element. Das Fixierungselement ist so konfiguriert, dass es in einem Hohlraum eines ersten Wirbelkörpers und gegen den Knochen des ersten Wirbelkörpers platziert wird.

Das aus WO 2012/045307 A1 bekannte Implantat umfasst einen die beiden Endplatten verbindenden rigiden Implantatträger und an der Peripherie des Implantatträgers angeordnete Strukturträger, welche vom Implantatträger weitgehend entkoppelt sind, aber mit dem Implantatträger im Bereich der Grenzflächen zum Knochen an mindestens einer Stelle verbunden sind. Die Strukturträger weisen eine Struktur in Form von regelmässig oder unregelmässig angeordneten Lamellen, Gittern, Ausformungen, Bohrungen oder Kavitäten auf. Wird das Implantat durch den Patienten belastet, führen die Strukturträger Relativbewegungen aus, wobei die Steifigkeit des Implantatträgers und die Anordnung der Strukturträger so gewählt sind, dass der durch die Strukturträger infolge einer Relativbewegung auf das Gewebe ausgeübte Dehnungsreiz zur beschleunigten Bildung von Knochenzellen führt.

Bei dieser bekannten Vorrichtung ist nachteilig, dass durch die Steifigkeit der Implantatträgers die Bewegung der Knochen vermindert wird, wodurch die angestrebte Vergrösserung der Dehnung kompromittiert wird.

Diese bekannte Vorrichtung hat somit spezielle Ziele, nämlich
- eine steife Verbindung der Endplatten herzustellen, welche der Kraftübertragung dient; sowie
- durch die Bewegung zwischen den mit den Endplatten verbundenen Strukturträgern den Dehnungsreiz zu erhöhen.

Wenn eine Kraft oder Moment über die Endplatten auf das Implantat einwirkt, verformt sich der Implantatträger, z. B. Längenänderung ΔL. Die Strukturträger sind fest mit der einen oder der anderen Endplatte verbunden und selbst rigide ausgebildet, so dass die Strukturträger selbst bei der Deformation des Implantatträgers keine nennenswerte
Deformation erfahren, d. h. die Lamellen oder Gitter werden um dasselbe Mass ΔL (absolute Längenänderung ΔL) relativ zueinander verschoben. Durch die Wahl des Abstands zwischen den Vertiefungen zweier einander gegenüberliegender Lamellen ist die lokale Dehnung des Gewebes, die aus der Relativverschiebung der Lamellen resultiert, in Kombination mit der Steifigkeit des Implantatträgers praktisch beliebig einstellbar.

Ein weiterer Nachteil dieses bekannten Implantats ist, dass der Stab zur Kraftübertragung zentral in der Konstruktion angebracht ist und somit an der Stelle, bei der sich in einem Röhrenknochen das Knochenmark befindet. Somit ist die Knochenmarkbildung bei diesem bekannten Implantat nicht möglich.

Hier will die Erfindung Abhilfe schaffen. Der Erfindung liegt die Aufgabe zugrunde, ein Knochenimplantat zu schaffen, welches auf Grund seiner Konstruktion, z. B. im Frakturspalt, zusammenhängende Bereiche optimaler Dehnung erzeugt. Dadurch wird z. B. eine solide Knochenbrücke von Knochenfragment zu Knochenfragment induziert und vermeidet damit die heilungsstörende, zu geringe oder zu grosse Dehnung.

Der Verkleinerung des aktiven Abstands der Knochenfragmente dienen Elemente, welche die zu überbrückende Strecke durch Parallelschaltung in kleinere Strecken unterteilen, wobei auf diesen kleineren Strecken eine grössere Dehnung ermöglicht wird.

Wenn kein erfindungsgemässes Implantat zwischen den Oberflächen der angrenzenden Knochenfragmente eingefügt ist, ergibt die Bewegung der Knochenfragment relativ zueinander der grossen Distanz zwischen den Knochenfragementen wegen eine geringe Dehnung. Ist die erwähnte Dehnung zu gering wird der Heilungsprozess nicht angeregt. Durch Einsetzen eines erfindungsgemässen Implantats zwischen die Knochenfragmente wird die gleiche Relativbewegung auf das zwischen den Gewebe-Aktivatoren angeordnete Knochenmaterial übertragen. Da die Gewebe-Aktivatoren den Gesamtzwischenraum zwischen den Knochenfragmenten in mehrere Längsabschnitte unterteilen, erfährt das Gewebe, welches in den Zwischenräumen zwischen je zwei Gewebe-Aktivatoren oder zwischen je einem Endstück und einem endständigen Gewebe-Aktivator angeordnet ist, wegen der gegenüber dem Frakturspalt geringeren Höhe dieser Zwischenräume eine grössere Dehnung, die den Heilungsprozess induziert und unterhält.

Die Erfindung löst die gestellte Aufgabe mit einem Implantat, welches die Merkmale des Anspruchs 1 aufweist.

Die durch die Erfindung gegenüber dem Stand der Technik erreichten Vorteile sind im Wesentlichen darin zu sehen, dass:
- die mit den Knochenfragmenten verbundenen Enden sich frei gegeneinander bewegen können, da diese nicht kraftschlüssig verbunden sind und somit die für die Heilung entscheidende Dehnung erhalten bleibt. Die mit den Enden jeweils verbundenen Aktivatoren stellen eine optimierte Dehnung im gesamten Bereich her;
- das erfindungsgemässe Implantat im Gegensatz zum Stand der Technik die Bildung von Knochenmark erlauben kann. Die Konstruktion erlaubt eine grosse Variabilität der Ausbildung der Aktivatoren inklusive der Ausbildung von Freiräumen an wesentlichen Stellen, durchgehend oder versetzt;
- im Gegensatz zum Stand der Technik die zwischen zwei Aktivatoren angeordneten und einer Dehnung unterworfenen Knochenmaterialpfropfen sich über die gesamte Querschnittsfläche des Knochens erstrecken, während bei dem bekannten Implantat nur das Gewebe an den Randbereichen des Implantats einem optimalen Dehnungsreiz unterworfen wird;
- die Aktivatoren im Gegensatz zu dem bekannten Implantat direkt mit den Endstücken verbunden sind und sich somit der dehnungsoptimierte Bereich von Ende zu Ende erstreckt; und
- die Aktivatoren im Gegensatz zu dem bekannten Implantat nicht durchgängig sind, zum Beispiel porös, und sich somit der Dehnungsoptimierte Bereich sich über mehrere Etagen erstreckt.

Die Erfindung kann überall dort angewendet werden, wo durch Korrektur oder Erzeugung der optimalen Dehnung Gewebsdifferenzierung angeregt oder ermöglicht
wird. Unter Anderem ist z.B. die Erzeugung von Weichgewebe oder Knochen für therapeutische oder ästetische Anwendungen Teil der Erfindung. Bei der Knochenverlängerung nach der Illizarov Methode wird heute in millimeter grossen Schritten verlängert da das Resultat eines grösseren Schrittes eine verkleinerte Dehnung bewirken würde um eine zuverlässige Knochenbildung zu erzeugen. Der Nachteil der erwähnten Methode besteht darin, das der Patient während langer Zeit die beschwerlichen externen Schienen erdulden muss. Durch Anwendung des erfindungsgemässen Implantats wird die für die Knochenbildung optimale Dehnung auch bei centimetergrossen Verlängerungsschritten erreicht. Die Tragzeit der den Patienten störenden Fixateure kann wesentlich verkürzt oder vermieden werden. Bei chirurgischen Eingriffen, die ein Transpalantation von autologem (körpereigenen) Knochen bedingen bewirkt die notwendige Knochenentnahme z.B. am Becken länger dauernde störende Schmerzen. Das erfindungsgeässe Implantat kann im Patienten eingesetzt die Knochenbildung erzeugen ohne die erwähnten schmerzhaften Folgen der Knochen-Entnahme.

Die durch die Erfindung erreichten Vorteile sind im wesentlichen darin zu sehen, dass dank dem erfindungsgemässen Implantat eine optimaler Dehnung erzeugt werden kann durch Veränderung des Abstands von Elementen, die der Bewegung der Knochenfragmente ausgesetzt sind.

Das erfindungsgemässe Implantat funktioniert bei allen Bewegungen, die das Gewebe deformieren, wie axialer Bewegung (Stauchung und Dehnung), Biegung, Torsion, paralleler Verschiebung, und anderen Bewegungen sowie bei Kombinationen von Bewegungs-Komponenten.

Das erfindungsgemässe Implantat kann dazu dienen an definierten Stellen die Heilung zu fördern. Er kann aber auch dazu dienen bestimmte Gewebe im Körper herzustellen, die generell für das "tissue engineering" eingesetzt werden.

Das erfindungsgemäss Implantat kann auch für die Knochenverlängerung eingesetzt werden. Es erlaubt solche Verlängerungen bis zu mehreren Zentimetern in einem einzigen Schritt. Dies ist der wesentliche Vorteil gegenüber der bekannten Illizarov Methode. Welche nur millimetergrosse Verlängerungsschritte gestattet. Mit dem erfindungsgemässen Implantat kann somit die Knochenverlängerung in einem oder wenigen Schritten erreicht werden. Der Patient ist kurz nach der Operation wieder voll funktionsfähig und das wochenlange Tragen bewegungsbehindernder externer Fixateur-Konstruktionen kann dadurch überflüssig werden.

Weitere vorteilhafte Ausgestaltungen der Erfindung können wie folgt kommentiert werden:
Die Ebenen der Gewebe-Aktivatoren können in verschiedenen Richtungen geneigt angeordnet sein.

Erfindungsgemäß sind mindestens zwischen zwei Gewebe-Aktivatoren ein oder mehrere federartige Elemente angeordnet.

In einer weiteren Ausführungsform weist das Implantat eine Umhüllende mit im Wesentlichen zylindrischer oder prismatischer Gestalt auf, wobei ein allfällig die Umhüllende bedeckender Mantel höchstens 40 %, vorzugsweise höchstens 20 % der gesamten Oberfläche der Umhüllenden ausmacht. Das aus dem Stand der Technik bekannte Implantat weist eine ausgedehnte zylindrische Mantelfläche auf, welche nur von gering dimensionierten Schlitzen oder Perforationen durchbrochen wird, so dass das einwachsen von Gewebe in das Implantat stark erschwert ist.

In einer weiteren Ausführungsform sind die Gewebe-Aktivatoren derart ausgestaltet und gegenseitig angeordnet, dass sie ein Einwachsen von Gewebe erlauben. Die Struktur der Gewebe-Aktivatoren soll ein solides Einwachsen von Blutgefässen und heilenden Geweben zwischen und innerhalb der Gewebe-Aktivatoren ermöglichen.

In einer anderen Ausführungsform sind die Gewebe-Aktivatoren plattenförmig, balkenartig, netzartig oder stabförmig ausgebildet. Die Gewebe-Aktivatoren können als vollständige Platten aber auch als Plattenteile ausgeführt werden z.B. Plattensegmente oder andere regelmässige oder unregelmässige Formen; die Plattenteile können Teile der Platte sein, die nebeneinander liegen, gestaffelt angeordnet oder vernetzt sind. Die Gewebe-Aktivatoren können auch so ausgebildet sein, dass diese eine zentrale Aussparung aufweisen.

In einer anderen Ausführungsform sind die Gewebe-Aktivatoren porös.

In wiederum einer anderen Ausführungsform sind die gegenüberliegenden Oberflächen der Gewebe-Aktivatoren planparallel, gebogen oder schräg ausgebildet. Die Oberflächen können verschiedenartig gewölbt oder gebogen sein, sodass die erwähnte Distanz von Ort zu Ort ändert und z.B. Stellen optimaler Dehnung erzeugt.

In einer weiteren Ausführungsform bestehen die Gewebe-Aktivatoren aus gewebeverträglichen Kunststoffen, Metallen, Metalllegierungen oder Keramik.

In einer anderen Ausführungsform bestehen die Gewebe-Aktivatoren aus bioresorbierbaren Materialien. Dadurch ist der Vorteil erreichbar, dass sich das Implantat mit der Zeit auflöst und verschwindet.

In einer anderen Ausführungsform umfassen die Oberfläche der Gewebe-Aktivatoren und/oder die Zwischenräume zwischen den Gewebe-Aktivatoren Substanzen, welche die Heilung und Knochenbildung fördern, vorzugsweise Bone Morphogenic Protein oder Knochenmehl.

In einer anderen Ausführungsform sind die Oberfläche der Gewebe-Aktivatoren und/oder die Zwischenräume zwischen den Gewebe-Aktivatoren mit Stammzellen versehen. Damit kann der Vorteil erreicht werden, dass dadurch die Heilung und Knochenbildung gefördert wird.

In wiederum einer anderen Ausführungsform sind die Zwischenräume zwischen den Gewebe-Aktivatoren mit einer offenporigen Struktur gefüllt.

In einer weiteren Ausführungsform sind das erste und zweite Endstück derart ausgebildet, dass sie eine kraftschlüssige mechanische Kopplung an die zwei Knochenfragmente gestatten, vorzugsweise (i) mechanisch mittels Pressfit, Verschraubung, Nagelung, Verplattung oder Heftung, oder (ii) chemisch mittels Verklebung.

In einer weiteren Ausführungsform ist die Verbindung der Gewebe-Aktivatoren mit den Endstücken mit einzelnen oder mehrteiligen Kraftträgern realisiert.

In einer anderen Ausführungsform sind die Kraftträger alternierend mit einem Gewebe-Aktivator fest verbunden oder durchqueren diesen ohne Kontakt. Die erwähnte alternierende Verbindung kann auch durch überbrückende Konstruktionen, die z.B. an den Peripherien der Aktivatoren ansetzen, ausgebildet werden.

In einer anderen Ausführungsform ist das erste Endstück mittels einem oder mehreren ersten Kraftträgern mit n > 1 ersten Gewebe-Aktivatoren verbunden und das zweite Endstück ist mittels einem oder mehreren zweiten Kraftträgern mit n > 1 zweiten Gewebe-Aktivatoren verbunden.

In einer weiteren Ausführungsform ist das erste Endstück mittels einem oder mehreren ersten Kraftträgern rigid mit den n > 1 ersten Gewebe-Aktivatoren verbunden.

In einer weiteren Ausführungsform ist das zweite Endstück mittels einem oder mehreren zweiten Kraftträgern rigid mit den n > 1 zweiten Gewebe-Aktivatoren verbunden.

Vorzugsweise ist das erfindungsgemässe Implantat geeignet zur Behandlung von Knochenfrakturen und Knochendefekten und zur Erzeugung von Knochengewebe.

Ein Verfahren zur Herstellung des erfindungsgemässen Implantats umfasst im Wesentlichen die folgenden Schritte:
A) Ermitteln der Länge des zu überbrückenden Frakturspalts;
B) Ermitteln der Anzahl Gewebe-Aktivatoren des Implantats aufgrund einer gewünschten Dehnung des zwischen den Gewebe-Aktivatoren eingefügten Gewebematerials; und
C) Herstellung eines Implantats mit der unter Schritt B) ermittelten Anzahl Gewebe- Aktivatoren. Die komplexe Struktur des Strain-Modifieres kann maschinell hergestellt werden z.B. durch 3D Druck z.B. des bioresorbierbaren Polylactids (PLA) aber auch durch CNC Fräsung, Laserschnitt, Schweissung, Wasserstrahlschnitt oder andere Verfahren, die Material auf- oder abtragen. Die Herstellung des erfindungsgemässen Implantats kann auch massgeschneidert mit Hilfe der oben erwähnten Verfahren hergestellt werden. Diese Herstellung kann im Rahmen des chirurgischen Eingriffs erfolgen.

Die Erfindung und Weiterbildungen der Erfindung werden im Folgenden anhand der teilweise schematischen Darstellungen mehrerer Beispiele noch näher veranschaulicht.

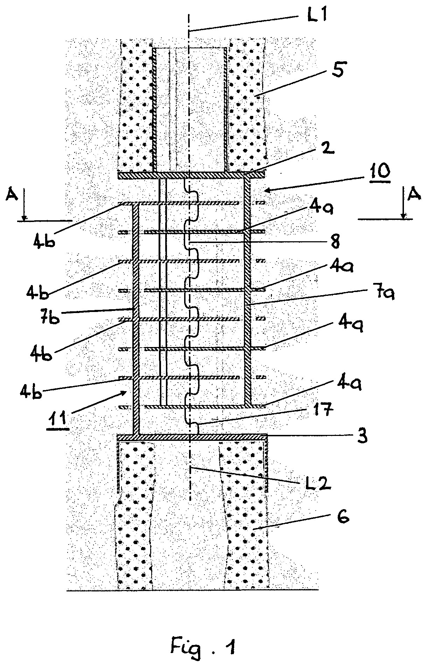

Die in den Figs. 1-4 gezeigten Implantate sind ohne federartige Elemente dargestellt und beschrieben. Erfindungsgemäß sind jedoch mindestens zwischen zwei Gewebe-Aktivatoren ein oder mehrere federartige Elemente angeordnet.

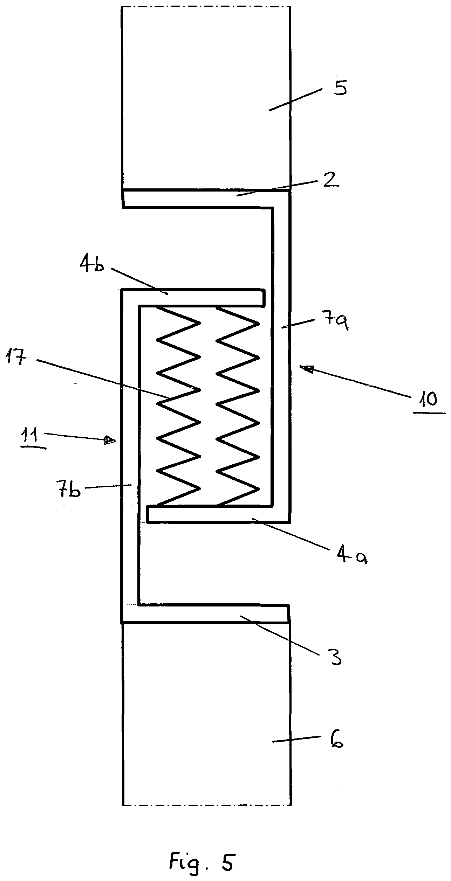

Das in Fig. 5 gezeigte Implantat ist mit nur je 1 Gewebe-Aktivator dargestellt und beschrieben.

Erfindungsgemäß stehen jedoch das erste und zweite Endstück mittels je n > 1 parallelgeschalteten Gewebe-Aktivatoren miteinander lose im Eingriff. Es zeigen:
Fig. 1 einen Längsschnitt durch eine Ausführungsform des Implantats;
Fig. 2 einen Schnitt längs der Linie A - A in Fig. 1 ;
Fig. 3 eine perspektivische Ansicht des Implantats gemäss Fig. 1 ;
Fig. 4 eine schematische Darstellung des zwischen zwei Knochenfragmenten eingesetzten Implantats gemäss Fig. 1 ; und
Fig. 5 eine schematische Darstellung einer weiteren Ausführungsform des Implantats.

Die in den Fig. 1 - 3 dargestellte Ausführungsform des Implantats 1 umfasst ein erstes Endstück 2, ein zweites Endstück 3 und mehrere Gewebe-Aktivatoren 4a;4b, welche alternierend mit je einem der Endstücke 2;3 verbunden sind, wobei eine erste Gruppe von Gewebe-Aktivatoren 4a in einer Parallelschaltung angeordnet ist und mit dem ersten Endstück 2 ein erstes Bauteil 10 bilden, welches an einem ersten Knochenfragment 5 befestigbar ist, und eine zweite Gruppe von Gewebe-Aktivatoren 4b ebenfalls in einer Parallelschaltung angeordnet ist und mit zweiten Endstück 3 ein zweites Bauteil 11 bilden, welches an einem zweiten Knochenfragment 6 befestigbar ist. Durch die alternierende Verbindung der Gewebe-Aktivatoren 4a;4b mit den Endstücken 2;3 wird eine Parallelschaltung erreicht, die die aktive Distanz, auf die sich die gegenseitige Bewegung der angrenzenden Knochenfragmente 5;6 auswirkt, so verkleinert, dass die Dehnung zwischen den Gewebe-Aktivatoren 4a;4b vergrössert wird. Insbesondere umfasst das Implantat 1 ein erstes Endstück 2 mit einer Längsachse L1, welches an ein erstes Knochenfragment 5 befestigbar ist, ein zweites Endstück 3 mit einer Längsachse L2, welches an ein zweites Knochenfragment 6 befestigbar ist, und zwei Gruppen von, beispielsweise aber nicht einschränkend, je vier parallelgeschalteten Gewebe-Aktivatoren 4a;4b, wobei jede Gruppe von Gewebe-Aktivatoren 4a;4b mit je einem der ersten und zweiten Endstücke 2;3 verbunden ist. Die Gewebe-Aktivatoren 4a;4b jeder der beiden Gruppen sind in Abständen zueinander angeordnet und greifen entlang einer Zentralachse 8 des Implantats 8 alternierend ineinander ein, derart, dass das erste und zweite Endstück 2;3 lose miteinander im Eingriff stehen, so dass eine Relativbewegung der ersten und zweiten Endstücke 2;3 gewährleistet ist. Die Gewebe-Aktivatoren 4a;4b sind quer, und insbesondere in der hier dargestellten Ausführungsform, beispielhaft aber nicht einschränkend, orthogonal zu den Längsachsen L1 und L2 der ersten und zweiten Endstücke 2;3 angeordnet. Die Längsachse L1 des ersten Endstücks 2 und die Längsachse L2 des zweiten Endstücks 3 sind in der hier dargestellten Ausführungsform des erfindungsgemässen Implantats 1 koaxial angeordnet und definieren eine Zentralachse 8 des Implantats 1, welche ebenfalls koaxial zu den Längsachsen L1, L2 angeordnet ist.

Die Gewebe-Aktivatoren 4a;4b übertragen die Bewegung der Endstücke 2;3 auf die Zwischenräume zwischen den erwähnten Gewebe-Aktivatoren 4a;4b und erhöhen so die Dehnung der durch Bewegung der Endstücke 2;3 bewirkten Deformation, die nun statt auf der ganzen Distanz zwischen den Endstücken 2;3 auf der kleineren Distanz zwischen den Gewebe-Aktivatoren 4a;4b wirkt. Damit wird die Dehnung des zwischen den Gewebe-Aktivatoren 4a;4b liegenden Materials erhöht, als Beispiel dient der schlecht heilende Knochen-Bruch oder -Defekt. Eine derartige Erhöhung der Dehnung ist für -das Bewirken der Heilung des Knochendefektes oder Knochenbruchs entscheidend.

Durch Serien-Schaltung eines Federelements 17 kann eine Reduktion der Dehnung zwischen den Gewebe-Aktivatoren 4a;4b [Dehnungsverminderung] erreicht werden. Das Federelement 17 kann, beispielhaft und nicht einschränkend, als Spiralfeder, gewundenes oder gefaltetes Element, in regelmässiger oder freier Form ausgeführt sein.

Alternativ können nur die Endstücke 2;3 mit dem jeweiligen Pakte von Gewebe-Aktivatoren 4a;4b verbunden aber nicht gegeseitig. Die Bewegung der Endstücke 2;3 wird dann nicht vermindert.

Das erste und zweite Endstück 2;3 sind im Wesentlichen derart ausgebildet, dass sie eine kraftschlüssige Kopplung an je eines der zwei Knochenfragmente 5;6 gestatten, wobei die folgenden Kopplungen oder Kombinationen davon besonders geeignet sind: (i) mechanisch mittels Presspassung, Verschraubung, Nagelung, Verplattung oder Heftung, oder (ii) chemisch mittles Verklebung.

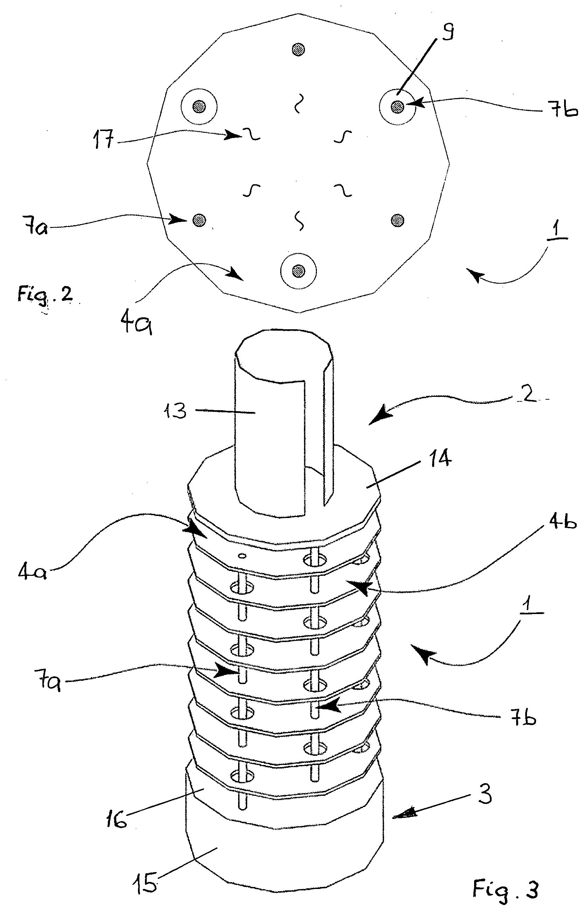

Fig. 3 zeigt eine perspektivische Ansicht dieser Ausführungsform des erfindungsgemässen Implantats 1 von schräg oben. Die Kraftträger 7a;7b sind alternierend mit den Gewebe-Aktivatoren 4a;4b verbunden oder queren diese ohne Verbindung. Beispielhaft ist die Verbindung der Gewebe-Aktivatoren 4a;4b mit den Knochen-Fragmenten 5;6 als geschlitzte Hülse, geschlitztes Rohr oder "Marknagel" ausgeführt, die einen Presssitz erlaubt. Das Implantat 1 kann aber auch ohne Verbindungselemente zwischen die Knochen-Fragmente 5;6 eingesetzt werden.

In der in den Fig. 1 ― 3 dargestellten Ausführungsform umfasst das erste Endstück 2, beispielhaft und nicht einschränkend, einen Längsabschnitt 13 in der Form eines geschlitzten Rohrs, welches im Markraum des ersten Knochenfragments 5 festklemmbar ist, und endständig an diesem Längsabschnitt 13 orthogonal zur Längsachse L1 angeordnet eine Endplatte 14 zur Anlage an die dem Frakturspalt zugewandte Stirnfläche des ersten Knochenfragments 5. Die Gewebe-Aktivatoren 4a der ersten Gruppe und das erste Endstück 2 sind mittels ersten Kraftträgern 7a untereinander verbunden und bilden ein erstes rigides Bauteil 10. Dabei sind die Endplatte 14 des ersten Endstücks 2 und der benachbarte Gewebe-Aktivator 4a und die entlang der Zentralachse 8 des Implantats 1 in gleichen Abständen zueinander angeordneten Gewebe-Aktivatoren 4a dieser ersten Gruppe miteinander, beispielhaft und nicht einschränkend, mittels drei ersten Kraftträgern 7a verbunden (Fig. 2).

Analog dazu umfasst zweite Endstück 3 umfasst einen Längsabschnitt 15 in der Form eines Rohrs, welches über die Aussenfläche des zweiten Knochenfragments 6 pressbar ist, und endständig an diesem Längsabschnitt 15 angeordnet eine Endplatte 16 zur Anlage an die dem Frakturspalt zugewandte Stirnfläche des zweiten Knochenfragments 6. Die Gewebe-Aktivatoren 4b der zweiten Gruppe und das zweite Endstück 3 sind mittels zweiten Kraftträgern 7b untereinander verbunden und bilden ein zweites rigides Bauteil 11. Dabei sind die Endplatte 16 des zweiten Endstücks 3 und der benachbarte Gewebe-Aktivator 4b und die entlang der Zentralachse 8 des Implantats 1 in gleichen Abständen zueinander angeordneten Gewebe-Aktivatoren 4b dieser zweiten Gruppe miteinander, beispielhaft und nicht einschränkend, ebenfalls mittels drei zweiten Kraftträgern 7b (Fig. 2) verbunden.

Die ersten und zweiten Kraftträger 7a;7b können als Stangen, Klammern, oder aus anderen Elementen ausgeführt werden, die die Gewebe-Aktivatoren 4a;4b alternierend mit den ersten und zweiten Endstücken 2;3 rigide verbinden. Dabei kann jeder der ersten und zweiten Kraftträger 7a;7b einstückig oder mehrteilig ausgebildet sein. Die Kraftträger 7a;7b erstrecken sich im Wesentlichen parallel zur Zentralachse 8 des Implantats und sind alternierend mit einem Gewebe-Aktivator 4a;4b fest verbunden oder durchqueren diesen ohne Kontakt. Alternativ können die Kraftträger 7a;7b an den Peripherien der Gewebe-Aktivatoren 4a;4b je einer Gruppe befestigt sein und aussen an der Peripherien der Gewebe-Aktivatoren 4a;4b der jeweils anderen Gruppe vorbeigeführt werden.

In der in den Fig. 1 - 3 dargestellten Ausführungsform des erfindungsgemässen Implantats 1 sind die Endplatten 14;16 der ersten und zweiten Endstücke 2;3 und die Gewebe-Aktivatoren 4a;4b plattenförmig mit einer polygonförmigen Peripherie ausgebildet. Wie in Fig. 2 ersichtlich, durchqueren je drei Kraftträger 7a;7b pro alternierendes Paket von Gewebe-Aktivatoren 4a;4b frei die Gewebe-Aktivatoren 4a;4b des einen Pakets und sind mit den Gewebe-Aktivatoren 4a;4b des anderen Pakets fest verbunden. Die Kraftträger 7a;7b jedes Bauteils 10;11 sind, beispielhaft und nicht einschränkend, in einem peripheren Bereich der Gewebe-Aktivatoren 4a;4b je in den Ecken eines gleichseitigen Dreiecks angeordnet, wobei die ersten und zweiten Kraftträger 7a;7b gleiche Abstände zueinander aufweisen. Dabei sind die drei Kraftträger 7a des ersten Bauteils 10 fest mit jedem Gewebe-Aktivator 4a der ersten Gruppe verbunden, während die drei Kraftträger 7b des zweiten Bauteils 11 durch entsprechend positionierte Bohrungen 9 in jedem der Gewebe-Aktivatoren 4a der ersten Gruppe durchgeführt werden. Analog dazu sind die drei Kraftträger 7b des zweiten Bauteils 10 fest mit jedem Gewebe-Aktivator 4b der zweiten Gruppe verbunden, während die drei Kraftträger 7a des ersten Bauteils 10 durch entsprechend positionierte Bohrungen 9 in jedem der Gewebe-Aktivatoren 4b der zweiten Gruppe durchgeführt werden. Alternativ können die Gewebe-Aktivatoren 4a;4b balkenartig, netzartig oder stabförmig ausgebildet sein. Die Gewebe-Aktivatoren 4a;4b können als vollständige Platten aber auch als Plattenteile ausgeführt werden, z.B. Plattensegmente oder andere regelmässige oder unregelmässige Formen, wobei die Plattenteile Teile einer Platte sein können, die nebeneinander liegen, gestaffelt angeordnet oder vernetzt sind.

Da beide Bauteile 10;11 rigid ausgebildet sind, d.h. die Gewebe-Aktivatoren 4a;4b jeder der zwei Gruppen fest mit dem entsprechenden Endstück 2;3 verbunden sind, wird eine beispielsweise auf das erste Knochenfragment 5 aufgebrachte Last über das erste Endstück 2 als Druckkraft auf das erste Bauteil 10 mit den Gewebe-Aktivatoren 4a der ersten Gruppe übertragen, so dass der Abstand zwischen der Endplatte 14 des ersten Endstücks 2 und dem endständigen Gewebe-Aktivator 4b des zweiten Bauteils 11 verkürzt wird und eine negative Dehnung oder Stauchung des dazwischen liegenden Knochenmaterials stattfindet, Zwischen dem endständigen Gewebe-Aktivator 4b des zweiten Bauteils 11 und dem ersten Gewebe-Aktivator 4a des ersten Bauteils 10, d.h. demjenigen Gewebe-Aktivator 4a der ersten Gruppe, der dem ersten Endstück 2 benachbart ist, wird hingegen der Abstand vergrössert, so dass eine positive Dehnung der eine Verlängerung des dazwischen liegenden Knochenmaterials stattfindet falls dieses an den Gewebe-Aktivatoren 4a;4b fixiert ist.

In verschiedenen Ausführungsformen des erfindungsgemässen Implantats 1 können die Gewebe-Aktivatoren 4a;4b porös sein. Ferner können die gegenüberliegenden Oberflächen der Gewebe-Aktivatoren 4a;4b planparallel, gebogen oder schräg ausgebildet sein, wobei die Oberflächen verschiedenartig gewölbt oder gebogen sein können, sodass der Zwischenraum zwischen je zwei Gewebe-Aktivatoren 4a;4b von Ort zu Ort ändert und z.B. Stellen optimaler Dehnung erzeugt. Die Gewebe-Aktivatoren 4a;4b sind aus gewebeverträglichen Kunststoffen, Metallen, Metalllegierungen oder Keramik hergestellt oder können auch aus bioresorbierbaren Materialien bestehen.

Vorzugsweise umfassen die Oberfläche der Gewebe-Aktivatoren 4a;4b und/oder die Zwischenräume zwischen den Gewebe-Aktivatoren 4a;4b Substanzen (nicht gezeichnet), welche die Heilung und Knochenbildung fördern, wobei sich insbesondere Bone Morphogenic Protein oder Knochenmehl eignet. Zu Förderung der Heilung und Knochenbildung können die Oberfläche der Gewebe-Aktivatoren 4a;4b und/oder die Zwischenräume zwischen den Gewebe-Aktivatoren 4a;4b mit Stammzellen versehen sein. Alternativ oder zusätzlich können die Zwischenräume zwischen den Gewebe-Aktivatoren 4a;4b mit einer offenporige Struktur gefüllt sein.

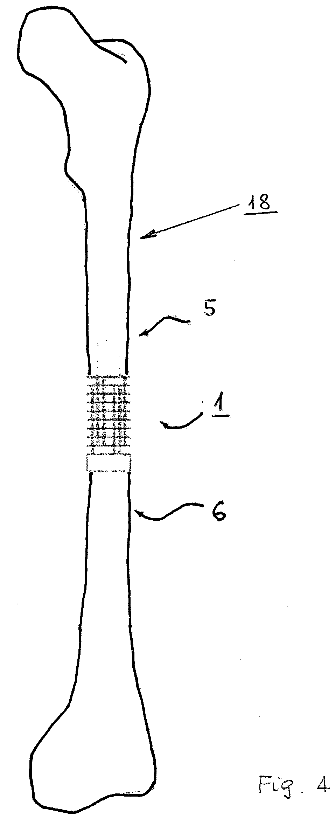

Das in Fig. 4 dargestellte erfindungsgemässe Implantat 1 dient in diesem Beispiel dazu ein schlecht oder nicht heilendes Segment eines Röhrenknochens 18, z.B. eines Femurs zwischen einem ersten und einem zweiten Knochenfragment 5;6 zu ersetzen. Andere Anwendungen sind nicht an dieses Beispiel des kortikalen Röhren-Knochens gebunden. Das erfindungsgemässe Implantat 1 kann auch in spongiösem Knochen und in Knochendefekten eingesetzt werden. Die durch Optimierung der Dehnung erzeugte Gewebedifferenzierung betrifft neben jener des Knochens auch die Differenzierung der Weichgewebe.

Die in Fig. 5 dargestellte Ausführungsform des erfindungsgemässen Implantats 1 unterscheidet sich von der in den Fig. 1 - 3 dargestellten Ausführungsform nur darin, dass das Implantat 1 beispielsweise und nicht einschränkend nur einen Gewebe-Aktivator 4a;4b pro Gruppe, d.h. pro Bauteil 10; 11 umfasst und dass zwischen den Gewebe-Aktivatoren 4a;4b Federelemente 17 eingesetzt sind. Wie oben beschrieben, liegt die optimale Gewebedehnung innerhalb einer Bandbreite zwischen einer zu geringen Dehnung, welche keine Heilung induziert und einer zu grossen Dehnung, welche keine Überbrückung des Bruchs durch Kallusbildung gestattet. Durch Letzteres würde eine solide Heilung verhindert. Das erfindungsgemässe Implantat 1 ermöglicht eine Erhöhung einer zu geringen Dehnung und ermöglicht durch die Federelemente 17 der in Fig. 4 dargestellten Ausführungsform auch eine Reduktion einer zu grossen Dehnung. Beispielhaft und nicht einschränkend sind zwei Federelemente 17 zwischen den Gewebe-Aktivatoren 4a;4b eingefügt. Alternativ können auch andere elastisch verformbare federartige Elemente eingesetzt werden.

Durch die Federelemente 17 wird einer auf die Knochenfragmente wirkenden Last ein zusätzlicher aber sehr geringer Widerstand entgegengebracht. Die Federelemente 17 dienen der Verteilung der Dehnung und haben keine Stützfunktion. Um Bereiche zu schaffen, welche einer unterschiedlichen Dehnung unterworfen sind, ist vorgesehen dass die Oberflächen der Aktivatoren 4a;4b gewölbt und nicht planparallel ausgeführt werden können. Die Gewebe-Aktivatoren 4a;4b können rigide oder nicht rigide, d.h. verschiebbar mit den Kraftträger 7a;7b verbunden sein. An den Kraftträgern 7a;7b können, z.B. endständig Arretierungen angeordnet sein, so dass die Gewebe-Aktivatoren 4a;4b mit den Kraftträgern 7a;7b verbunden bleiben.

Wie bereits oben erwähnt liegt die optimale Gewebedeformation (Dehnung) innerhalb einer Bandbreite zwischen zu geringer Dehnung, bei welcher eine Heilung nicht induziert wird, und einer zu grossen Dehnung, welche keine Überbrückung des gebildeten Kallus ermöglicht. Die Letztere gestattet keine solide Heilung. Das erfindungsgemässe Implantat ermöglicht eine zu geringe Dehnung zu vergrössern, ist aber auch dazu geeignet, eine zu grosse Dehnung zu reduzieren aufgrund der folgenden Eigenschaft: Zwischen den Gewebe-Aktivatoren 4a;4b werden federähnliche Elemente oder Federelemente 17 eingefügt. Entlang diesen Federelementen 17, welche den Abstand zwischen den Gewebe-Aktivatoren 4a;4b erheblich vergrössern, wird das Gewebe einer reduzierten Deformation ausgesetzt, weil für dieselbe Verschiebung die Distanz, über welche das Gewebe deformiert wird, mehrfach vergrössert wird. Dadurch wird die Grösse der Gewebedeformation reduziert. Der Effekt dieser Vergrösserung der Distanz aufgrund der Verwendung von Federelementen 17 ist um ein mehrfaches grösser als der Effekt einer kürzeren Distanz zwischen den Gewebe-Aktivatoren 4a;4b, welcher die Dehnung vergrössern würde. Somit werden in einem Implantat 1 Bereiche mit grösserer Dehnung (direkt verbunden zwischen den Gewebe-Aktivatoren 4a;4b) und Bereiche mit kleinerer Dehnung entlang den Federelementen 17 eingerichtet.

Das Gewebe, welches eine optimale Dehnung erfährt, entweder zwischen den Gewebe-Aktivatoren 4a;4b oder entlang den Federelementen 17 ermöglicht die Heilung, wobei es für eine korrekte Funktion nicht erforderlich ist, dass überall dieselbe Dehnungsbedingungen vorhanden sind. Ausgehend von den Bereichen mit optimaler Dehnung beginnt die Heilung und das nachfolgende feste Überbrücken reduziert die Verschiebung zwischen den Fragmentenden, respektive zwischen den Enden des Implantats 1, so dass eine Heilung über dem gesamten Querschnitt ermöglicht wird.

Diese Erfindung ist nicht auf die oben erwähnten, besonders bevorzugten Ausführungsformen beschränkt. Der Schutzbereich der Erfindung wird durch die Patentansprüche definiert.

## Patentansprüche

1. Implantat (1) zur Modifikation der Gewebedeformation während der Knochenheilung zwischen zwei Knochenfragmenten (5; 6), mit
(i) einem ersten, an einem ersten Knochenfragment (5) befestigbaren Endstück (2) mit einer Längsachse L1;
(ii) einem zweiten, an einem zweiten Knochenfragment (6) befestigbaren Endstück (3) mit einer Längsachse L2;
(iii) Gewebe-Aktivatoren (4a; 4b), die den Gesamtzwischenraum zwischen den Knochenfragmenten in mehrere Längsabschnitte unterteilen, wodurch das Gewebe, welches in den Zwischenräumen zwischen je zwei Gewebe-Aktivatoren oder zwischen je einem Endstück und einem endständigen Gewebe-Aktivator angeordnet ist, wegen der gegenüber dem Frakturspalt geringeren Höhe dieser Zwischenräume eine größere Dehnung erfährt, die den Heilungsprozess induziert;
wobei die Gewebe-Aktivatoren (4a; 4b) alternierend mit je einem der Endstücke (2; 3) verbunden sind;
wobei eine erste Gruppe von Gewebe-Aktivatoren (4a) in einer Parallelschaltung angeordnet ist und mit dem ersten Endstück (2) ein erstes Bauteil (10) bilden, welches an dem ersten Knochenfragment (5) befestigbar ist, und eine zweite Gruppe von Gewebe-Aktivatoren (4b) ebenfalls in einer Parallelschaltung angeordnet ist und mit dem zweiten Endstück (3) ein zweites Bauteil (11) bilden, welches an dem zweiten Knochenfragment (6) befestigbar ist; wobei das erste und zweite Endstück (2; 3) mittels je n > 1 parallelgeschalteten Gewebe-Aktivatoren (4a; 4b) miteinander lose im Eingriff stehen und eine Relativbewegung zulassen; wobei die Gewebe-Aktivatoren (4a; 4b) quer zu den Längsachsen L1 und L2 und vorzugsweise orthogonal dazu angeordnet sind;
**dadurch gekennzeichnet, dass**
mindestens zwischen zwei Gewebe-Aktivatoren (4a; 4b) ein oder mehrere federartige Elemente (17) angeordnet sind.

2. Implantat (1) nach Anspruch 1, **dadurch gekennzeichnet, dass** das Implantat (1) eine Umhüllende mit im Wesentlichen zylindrischer oder prismatischer Gestalt aufweist und dass ein allfällig die Umhüllende bedeckender Mantel höchstens 40 %, vorzugsweise höchstens 20 % der gesamten Oberfläche der Umhüllenden ausmacht.

3. Implantat (1) nach einem der Ansprüche 1 bis 2, **dadurch gekennzeichnet, dass** die Gewebe-Aktivatoren (4a; 4b) derart ausgestaltet und gegenseitig angeordnet sind, dass sie ein Einwachsen von Gewebe erlauben.

4. Implantat (1) nach Anspruch 3, **dadurch gekennzeichnet, dass** die Gewebe-Aktivatoren (4a; 4b) plattenförmig, balkenartig, netzartig oder stabförmig ausgebildet sind.

5. Implantat (1) nach Anspruch 3 oder 4, **dadurch gekennzeichnet, dass** die Gewebe-Aktivatoren (4a; 4b) porös sind.

6. Implantat (1) nach einem der Ansprüche 1 bis 5, **dadurch gekennzeichnet, dass** die gegenüberliegenden Oberflächen der Gewebe-Aktivatoren (4a; 4b) planparallel, gebogen oder schräg ausgebildet sind.

7. Implantat (1) nach einem der Ansprüche 1 bis 6, **dadurch gekennzeichnet, dass** die Gewebe-Aktivatoren (4a; 4b) aus gewebsverträglichen Kunststoffen, Metallen, Metalllegierungen oder Keramik bestehen.

8. Implantat (1) nach einem der Ansprüche 1 bis 6, **dadurch gekennzeichnet, dass** die Gewebe-Aktivatoren (4) aus bioresorbierbaren Materialien bestehen.

9. Implantat (1) nach einem der Ansprüche 1 bis 8, **dadurch gekennzeichnet, dass** die Oberfläche der Gewebe-Aktivatoren (4a; 4b) und/oder die Zwischenräume zwischen den Gewebe-Aktivatoren (4a; 4b) Substanzen umfassen, welche die Heilung und Knochenbildung fördern, vorzugswiese Bone Morphogenic Protein oder Knochenmehl.

10. Implantat (1) nach einem der Ansprüche 1 bis 8, **dadurch gekennzeichnet, dass** die Oberfläche der Gewebe-Aktivatoren (4a; 4b) und/oder die Zwischenräume zwischen den Gewebe-Aktivatoren (4a; 4b) mit Stammzellen versehen sind.

11. Implantat (1) nach einem der Ansprüche 1 bis 10, **dadurch gekennzeichnet, dass** die Zwischenräume zwischen den Gewebe-Aktivatoren (4a; 4b) mit einer offenporigen Struktur gefüllt sind.

12. Implantat (1) nach einem der Ansprüche 1 bis 11, **dadurch gekennzeichnet, dass** das erste und zweite Endstück (2; 3) derart ausgebildet sind, dass sie eine kraftschlüssige mechanische Kopplung an die zwei Knochenfragmente gestatten, vorzugswiese (i) mechanisch mittels Pressfit, Verschraubung, Nagelung, Verplattung oder Heftung, oder (ii) chemisch mittles Verklebung.

13. Implantat (1) nach einem der Ansprüche 1 bis 12, **dadurch gekennzeichnet, dass** die Verbindung der Gewebe-Aktivatoren (4a; 4b) mit den Endstücken (2; 3) mit einzelnen oder mehrteiligen Kraftträgern (7a; 7b) realisiert ist und die Kraftträger (7a; 7b) alternierend mit einem Gewebe-Aktivator (4a; 4b) fest verbunden sind oder diesen ohne Kontakt durchqueren.

14. Implantat (1) nach Anspruch 13, **dadurch gekennzeichnet, dass** das erste Endstück (2) mittels einem oder mehreren ersten Kraftträgern (7a) rigid mit den n > 1 ersten Gewebe-Aktivatoren (4a) verbunden ist, und das zweite Endstück (3) mittels einem oder mehreren zweiten Kraftträgern (7b) rigid mit den n > 1 zweiten Gewebe-Aktivatoren (4b) verbunden ist.

15. Implantat (1) gemäß einem der Ansprüche 1 bis 14, geeignet zur Behandlung von Knochenfrakturen und Knochendefekten und zur Erzeugung von Knochengewebe.

16. lmplantat gemäß einem der Ansprüche 1 bis 15, geeignet zur Erzeugung von Weichgewebe oder Knochen für therapeutische oder ästhetische Anwendungen.

## Claims

1. Implant (1) for modifying tissue deformation during bone healing between two bone fragments (5; 6), comprising:
(i) a first end piece (2) with a longitudinal axis L1, which can be fastened to a first bone fragment (5);
(ii) a second end piece (3) with a longitudinal axis L2, which can be fastened to a second bone fragment (6);
(iii) tissue activators (4a; 4b) which divide the total space between the bone fragments into several longitudinal sections, as a result of which the tissue arranged in the spaces between two tissue activators or between an end piece and a terminal tissue activator undergoes greater expansion due to the smaller height of these spaces relative to the fracture gap, which induces the healing process;
wherein the tissue activators (4a; 4b) are alternately connected to one of the end pieces (2; 3) respectively;
wherein a first group of tissue activators (4a) is arranged in a parallel connection and forms with the first end piece (2) a first component (10) which can be fastened to the first bone fragment (5), and a second group of tissue activators (4b) is also arranged in a parallel connection and forms with the second end piece (3) a second component (11) which can be fastened to the second bone fragment (6);
wherein the first and second end pieces (2; 3) are loosely engaged with one another via n > 1 tissue activators (4a; 4b) connected in parallel and allow a relative movement; wherein the tissue activators (4a; 4b) are arranged transversely to the longitudinal axes L1 and L2 and preferably orthogonally thereto;
**characterised in that**
one or more spring-like elements (17) are arranged at least between two tissue activators (4a; 4b).

2. Implant (1) according to claim 1, **characterised in that** the implant (1) has a wrapping in an essentially cylindrical or prismatic shape, and **in that** a jacket that may cover the wrapping accounts for a maximum of 40%, preferably a maximum of 20%, of the total surface area of the wrapping.

3. Implant (1) according to any one of claims 1 to 2, **characterised in that** the tissue activators (4a; 4b) are designed and arranged relative to one another in such a way that they allow the ingrowth of tissue.

4. Implant (1) according to claim 3, **characterised in that** the tissue activators (4a; 4b) are designed to be plate-shaped, bar-like, mesh-like or rod-shaped.

5. Implant (1) according to claim 3 or 4, **characterised in that** the tissue activators (4a; 4b) are porous.

6. Implant (1) according to any one of claims 1 to 5, **characterised in that** the opposing surfaces of the tissue activators (4a; 4b) are plane-parallel, curved or oblique.

7. Implant (1) according to any one of claims 1 to 6, **characterised in that** the tissue activators (4a; 4b) consist of tissue-compatible plastics, metals, metal alloys or ceramic.

8. Implant (1) according to any one of claims 1 to 6, **characterised in that** the tissue activators (4) consist of bioresorbable materials.

9. Implant (1) according to any one of claims 1 to 8, **characterised in that** the surface of the tissue activators (4a; 4b) and/or the spaces between the tissue activators (4a; 4b) contain substances that promote healing and bone building, preferably bone morphogenic protein or bone meal.

10. Implant (1) according to any one of claims 1 to 8, **characterised in that** the surface of the tissue activators (4a; 4b) and/or the spaces between the tissue activators (4a; 4b) are provided with stem cells.

11. Implant (1) according to any one of claims 1 to 10, **characterised in that** the spaces between the tissue activators (4a; 4b) are filled with an open-pore structure.

12. Implant (1) according to any one of claims 1 to 11, **characterised in that** the first and second end pieces (2; 3) are designed in such a way that they allow a force-fit mechanical coupling to the two bone fragments, preferably (i) mechanically by means of press fitting, screwing, nailing, plating or tacking, or (ii) chemically by means of adhesive bonding.

13. Implant (1) according to any one of claims 1 to 12, **characterised in that** the connection of the tissue activators (4a; 4b) to the end pieces (2; 3) is created with individual or multi-part load-bearing elements (7a; 7b) and the load-bearing elements (7a; 7b) are alternately firmly connected to a tissue activator (4a; 4b) or cross over it without contact.

14. Implant (1) according to claim 13, **characterised in that** the first end piece (2) is rigidly connected to the n > 1 first tissue activators (4a) by means of one or more first load-bearing elements (7a), and the second end piece (3) is connected to the n > 1 second tissue activators (4b) by means of one or more second load-bearing elements (7b).

15. Implant (1) according to any one of claims 1 to 14, suitable for the treatment of bone fractures and bone defects and for generating bone tissue.

16. Implant according to any one of claims 1 to 15, suitable for generating soft tissue or bones for therapeutic or aesthetic applications.

## Revendications

1. Implant (1) permettant de modifier la déformation tissulaire lors de la consolidation osseuse entre deux fragments osseux (5 ; 6), comportant
(i) un premier embout (2) pouvant être fixé sur un premier fragment osseux (5) et présentant un axe longitudinal L1 ;
(ii) un second embout (3) pouvant être fixé sur un second fragment osseux (6) et présentant un axe longitudinal L2 ;
(iii) des activateurs tissulaires (4a ; 4b), lesquels divisent l'espace entre les fragments osseux en plusieurs sections longitudinales, le tissu, lequel est disposé dans les espaces entre deux activateurs tissulaires ou entre chaque embout et un activateur tissulaire terminal, en raison de la hauteur inférieure de ces espaces par rapport au trait de fracture, subissant un étirement plus important, lequel induit le processus de consolidation ;
dans lequel les activateurs tissulaires (4a ; 4b) sont respectivement reliés alternativement à l'un des embouts (2 ; 3) ;
dans lequel un premier groupe d'activateurs tissulaires (4a) est disposé selon une connexion en parallèle et forme avec le premier embout (2) un premier composant (10), lequel peut être fixé au premier fragment osseux (5), et un second groupe d'activateurs tissulaires (4b) est également disposé selon une connexion en parallèle et forme avec le second embout (3) un second composant (11), lequel peut être fixé au second fragment osseux (6) ;
dans lequel les premier et second embouts (2 ; 3) sont en prise lâche l'un avec l'autre au moyen de n > 1 activateurs tissulaires (4a ; 4b) connectés en parallèle et permettent un mouvement relatif ; dans lequel les activateurs tissulaires (4a ; 4b) sont disposés transversalement aux axes longitudinaux L1 et L2 et, de préférence orthogonalement ;
**caractérisé en ce que**
un ou plusieurs éléments (17) en forme de ressort sont disposés au moins entre deux activateurs tissulaires (4a ; 4b).

2. Implant (1) selon la revendication 1, **caractérisé en ce que** l'implant (1) comporte une extrémité enveloppante de forme essentiellement cylindrique ou prismatique et **en ce que** toute enveloppe recouvrant éventuellement l'extrémité enveloppante représente au plus 40 %, de préférence au plus 20 %, de la surface totale de l'extrémité enveloppante.

3. Implant (1) selon l'une quelconque des revendications 1 à 2, **caractérisé en ce que** les activateurs tissulaires (4a ; 4b) sont conçus et disposés de manière opposée l'un à l'autre pour permettre la croissance des tissus.

4. Implant (1) selon la revendication 3, **caractérisé en ce que** les activateurs tissulaires (4a ; 4b) sont en forme de plaque, en forme de barre, en forme de filet ou en forme de tige.

5. Implant (1) selon la revendication 3 ou 4, **caractérisé en ce que** les activateurs tissulaires (4a ; 4b) sont poreux.

6. Implant (1) selon l'une quelconque des revendications 1 à 5, **caractérisé en ce que** les surfaces opposées des activateurs tissulaires (4a ; 4b) sont parallèles, courbées ou inclinées.

7. Implant (1) selon l'une quelconque des revendications 1 à 6, **caractérisé en ce que** les activateurs tissulaires (4a ; 4b) sont constitués de matières plastiques, de métaux, d'alliages métalliques ou de céramiques compatibles avec les tissus.

8. Implant (1) selon l'une quelconque des revendications 1 à 6, **caractérisé en ce que** les activateurs tissulaires (4) sont constitués de matières biorésorbables.

9. Implant (1) selon l'une quelconque des revendications 1 à 8, **caractérisé en ce que** la surface des activateurs tissulaires (4a ; 4b) et/ou les espaces entre les activateurs tissulaires (4a ; 4b) comportent des substances, lesquelles favorisent la consolidation et la formation osseuse, de préférence une protéine morphogénétique osseuse ou une poudre d'os.

10. Implant (1) selon l'une quelconque des revendications 1 à 8, **caractérisé en ce que** la surface des activateurs tissulaires (4a ; 4b) et/ou les espaces entre les activateurs tissulaires (4a ; 4b) sont pourvus de cellules souches.

11. Implant (1) selon l'une quelconque des revendications 1 à 10, **caractérisé en ce que** les espaces entre les activateurs tissulaires (4a ; 4b) sont remplis d'une structure à pores ouverts.

12. Implant (1) selon l'une quelconque des revendications 1 à 11, **caractérisé en ce que** les premier et second embouts (2 ; 3) sont conçus de telle sorte qu'ils permettent un accouplement mécanique par complémentarité de force sur les deux fragments osseux, de préférence (i) mécaniquement par ajustement serré, vissage, enclouage, placage ou agrafage, ou (ii) par collage chimique.

13. Implant (1) selon l'une quelconque des revendications 1 à 12, **caractérisé en ce que** la connexion des activateurs tissulaires (4a ; 4b) avec les embouts (2 ; 3) est réalisée avec des supports de force (7a ; 7b) individuels ou en plusieurs parties et les supports de force (7a ; 7b) sont alternativement reliés fermement à un activateur tissulaire (4a ; 4b) ou le traversent sans contact.

14. Implant (1) selon la revendication 13, **caractérisé en ce que** le premier embout (2) est relié rigidement aux n > 1 premiers activateurs tissulaires (4a) au moyen d'un ou de plusieurs premiers supports de force (7a), et le second embout (3) est relié rigidement aux n > 1 seconds activateurs tissulaires (4b) au moyen d'un ou de plusieurs seconds supports de force (7b).

15. Implant (1) selon l'une quelconque des revendications 1 à 14, adapté au traitement des fractures osseuses et des défauts osseux et pour produire du tissu osseux.

16. Implant selon l'une quelconque des revendications 1 à 15, adapté à la production du tissu mou ou d'os pour des applications thérapeutiques ou esthétiques.
